# EUROPEAN PATENT SPECIFICATION

(11) **EP 1 354 567 B1**
(45) Date of publication and mention of the grant of the patent: **29.10.2008**
(21) Application number: 02008656.7
(22) Date of filing: 17.04.2002
(51) Int. Cl.: A61C 13/00, A61C 5/10, A61K 6/04, B22F 3/11, B22F 3/26, C22C 1/04

(54) **Method of forming a dental coping**
Verfahren zur Herstellung von Zahnhülse
Procédé de formation d'un chaperon dentaire

(43) Date of publication of application: 22.10.2003
(73) Proprietor: Shoher, Itzhak, Dr., Tel Aviv 61580 (IL); Whiteman, Aharon, Tel Aviv 61580 (IL)
(72) Inventor: Shoher, Itzhak, Dr., Tel Aviv 61580 (IL); Whiteman, Aharon, Tel Aviv 61580 (IL)
(74) Representative: Kinzebach, Werner

(56) References cited:
- WO-A-01/50975
- US-A- 5 336 091
- US-A- 6 027 012
- US-B1- 6 382 980

## Description

### FIELD OF THE INVENTION

This invention relates to a method of forming a dental coping in a single heat treatment from a composite material having at least two layers and to a composite material formed from laminated multiple metallic layers in an alternating arrangement for use in forming a dental crown or bridge.

### BACKGROUND OF THE INVENTION

A metal coping is used in dentistry in the construction of a dental crown and/or bridge. The metal coping functions as the under structure of the crown and is usually covered, for reasons of aesthetics, with a fired-on coating of a ceramic porcelain composition or a polymer based veneering material. The metal coping supports the coating and provides the required structural strength and rigidity for the restored tooth to resist the forces of mastication.

A metal coping may be cast from an investment of a wax or plastic pattern of the tooth to be restored. An alternative procedure which does not require waxing, investing or casting and which currently has been gaining wide acceptance by many laboratory practitioners and dentists is to form the coping from a moldable dental material composition composed of a mixture of high and low fusing temperature metal particles, as disclosed, for example, in U.S. Patent No. 5,234,343 and US Patent No. 5,332,622 respectively. The dental material as taught in these patents forms a porous structure upon heat treatment having a high void volume of above at least 20%. Before heat treatment the dental material is molded into the shape of the tooth to be restored. The molded shape is self-supported and is converted upon heat treatment in a dental furnace into a porous structure essentially any without shrinkage. The heat treatment temperature must be sufficient to entirely or substantially melt the low fusing temperature metal particles but not the high fusing temperature metal particles. This results in the low fusing temperature metal particles interconnecting the high fusing temperature metal particles to form the porous structure without affecting the shape of the structure. The porous structure has a high void volume which is then filled to solidify the structure by adding a filler material of a metal or ceramic in a secondary heat treatment procedure.

In US Patent No. 5,593,305, it is further taught that the moldable dental material may be formed into a compacted strip formed of a base material of the high and low fusing temperature metal particles and wax. Likewise the filler material may also be in a compacted strip of the filler composition and wax. The strip of base material is hand molded over the surface of a die and heat treated followed by a similar procedure for the strip of filler material in a secondary heat treatment operation.

WO 0 150 975 A describes a method for manufacturing dental restorations wherein a metal powder of a high fusing metal is applied to a die covered by a flowable paste and sprinkled by a second covering material.

### SUMMARY OF THE INVENTION

It has been discovered in accordance with the present invention that a coping formed of two or more superimposed metallic layers of high and low fusing temperature metal particle compositions can be heat treated in a dental furnace in a single operation with the heat treatment carried out gradually or in stages either manually or automatically. Heretofore, it was necessary to heat treat the base metallic layer of high fusing temperature metal particles separately to form an independent porous structure before adding filler material. The filler material was applied to the porous structure followed by a separate heat treatment operation to cause the porous structure to densify.

The method for forming a dental coping in accordance with the present invention comprises the steps of: forming a first sheet composed of a base material composition containing high fusing temperature metal particles and a wax binder; forming a second sheet of a filler material composition containing low fusing temperature metal particles and a wax binder, superimposing the two sheets to form a laminate, molding the sheets either separately or as a laminate over the surface of a die into the shape of a tooth to be restored, placing the molded laminate into a furnace and raising the temperature in the furnace from a temperature substantially below the melting temperature of the low temperature fusing particles to a maximum temperature not to exceed 1075°C. The furnace temperature should preferably be raised gradually or in stages and over a time period sufficient to cause the high fusing temperature metal particles to form a stable porous structure of high fusing temperature metal before a temperature is reached in the furnace sufficient to melt and densify the low fusing particles into the porous structure.

The composite material of the present invention is formed from superimposed metallic layers in an alternating arrangement for use in forming a dental crown or bridge. The material should preferably comprise at least three layers with at least one of the layers composed of a base material composition containing high fusing temperature metal particles and with one other layer adjacent thereto comprising a filler material composition of low fusing temperature metal particles with said layers arranged in an alternating sequence for forming a dental coping upon firing the multi-layer structure in a dental furnace at a temperature at least equal to the melting temperature of the low fusing temperature metal particles but below the melting temperature of the high fusing temperature metal particles. The dental material of the present invention is adapted to solidify upon heat treatment with the layers of filler material merging into the layers of the base material. The alternating arrangement of layers, the composition of the layers and the thickness of the layers control the degree of diffusion of the filler material into the layer(s) of base material. The diffusion occurs substantially simultaneous with the conversion of the base layers) into a porous structure. The layer(s) of filler material merge into the porous structure formed by the base material during heat treatment and solidify into a dental coping. The physical properties of the dental coping following heat treatment such as e.g., its hardness can be modified by variation of the composition and/or thickness of the layers. The arrangement of layers and their composition may also be varied to improve the adaptability of the surface layer to a fired-on coating of porcelain and/or to improve its adaptability to cementation.

### BRIEF DESCRIPTION OF THE DRAWINGS

Additional advantages of the present invention will become apparent from the following detailed description of the invention when read in conjunction with the accompanying drawings of which:
Figure 1 is a cross sectional view of one embodiment of the dental material of the present invention shown molded over a dental die of a tooth to be restored before heat treatment;
Figure 1a is an enlargement of the laminated layers of the dental material of Figure 1;
Figure 2 is a cross sectional view of the solidified dental material structure of Figure 1 after heat treatment but before removal from the die;
Figure 3 is a cross sectional view of a laminated three layer composite of the dental material of the present invention before heat treatment;
Figure 3a is a cross sectional view of the solidified structure of Figure 3 after heat treatment;
Figure 4 is a cross sectional view of an alternate arrangement of a laminated three layer composite of the dental material of the present invention before heat treatment;
Figure 4a is a cross sectional view of the solidified structure of Figure 4 after heat treatment;
Figure 5 is another cross sectional view of an alternate arrangement of a laminated five layer composite of the dental material of the present invention before heat treatment;
Figure 5a is a cross sectional view of the solidified structure of Figure 5 after heat treatment;
Figure 6 is yet another cross sectional view of an alternate arrangement of a laminated five layer composite of the dental material of the present invention before heat treatment; and
Figure 6a is a cross sectional view of the solidified structure of Figure 5 after heat treatment.

### DETAILED DESCRIPTION OF THE INVENTION

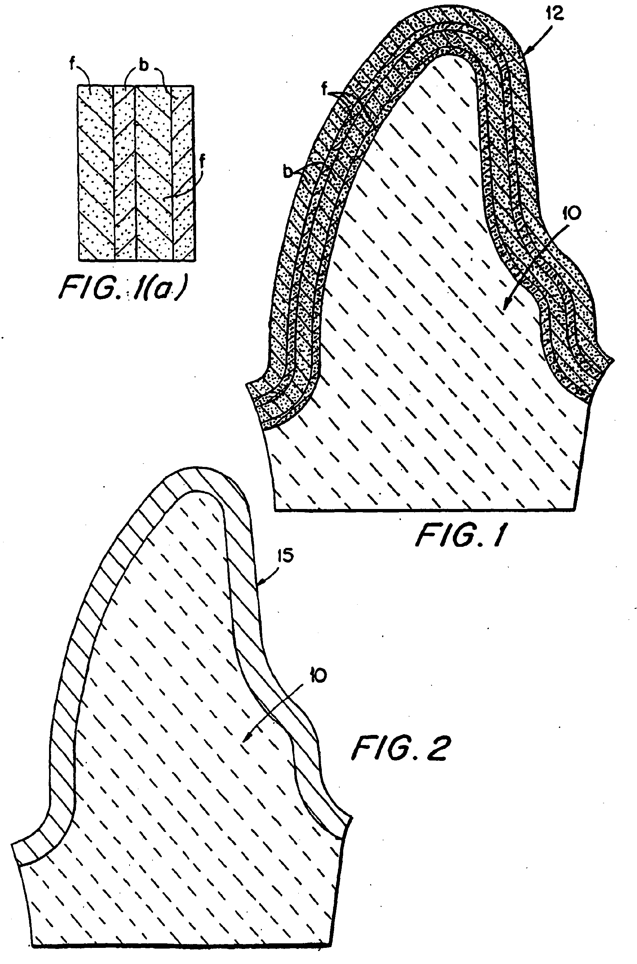

Referring now to Figures 1 and 2 showing a dental die 10 in cross section having an external shape conforming to the tooth to be restored upon which is molded the dental material 12 of the present invention. The dental material 12 is a laminated structure having a minimum of two layers composed of different metallic particle compositions identified hereafter as "b" and "f" respectively although three or more layers is preferred. The structure 12 as shown in Figures 1(a) and 2 has, for purpose of illustration, four layers two of which are composed of metallic particle compositions "b" and "f" which preferably alternate to form the four layers. The layer having composition "b" constitutes the base material and is of a composition containing high fusing temperature metal particles in an aggregate mixture with a volatile binder. The volatile binder is preferably a wax composition which holds the mixture together. The base material may also contain low fusing temperature metal particles. The concentration of the binder may vary widely although preferably between twenty percent by volume and up to eighty percent by volume of the base material composition. A wax should be selected which is relatively soft and tacky to form the binder and may be selected from any natural wax, mineral wax, or organic wax composition. The binder should melt relatively cleanly without leaving a significant residue at a melting temperature well below or at least no higher than the melting temperature of the low-fusing temperature metal particles.

The base material layer "b" of high-fusing temperature metal particles may be selected from a single metal or metal alloy, preferably of precious metals such as platinum and palladium in any desired proportion relative to one another from zero to one hundred percent. Additional constituents may be added such as gold, silver, copper, magnesium, aluminum, zinc, gallium, indium and other metals selected from the third, fourth or fifth group of elements of the periodic table. The total weight percent of the elements other than gold, silver, and the platinum group metals should not exceed ten percent. Gold may be added in any proportion to the high-fusing temperature metal component to increase the affinity of the high-fusing temperature metal component to the low-fusing temperature metal components or to itself in the absence of other low fusing component. In the latter instance gold may represent a major constituent of the high fusing metal composition and depending upon its concentration may substantially lower the melting temperature of the high fusing component.

The layer "f' constitutes a filler material composition of low fusing temperature metal particles composed preferably of gold or a gold alloy, with gold as the major constituent. The preference for gold as the major constituent of the low-fusing filler material "f" is based on its known characteristics of workability, biocompatibility, non-oxidizing properties and color. The layer of filler material "f" must possess a melting temperature below that of the high fusing temperature metal particles in the base material composition.

In Figure 1, and preferably as shown enlarged in Figure 1a, the layers "b" and "f" lie adjacent to one another in an alternating arrangement with the filler material layer "f' selected as the outer layer of the composite in a tandem arrangement of layers to form a composite laminated arrangement with two "b" layers and two "f" layers. However, it should be understood that any number of layers above two can be forme. An arrangement of at least three alternating layers is preferable.

A dental coping 15 is formed, as shown in Figure 2. when the dental material 12 of Figure 1 is subjected to heat treatment in a dental furnace at a temperature at least equal to the melting temperature of the low fusing temperature metal particles of the filler material composition but below the melting temperature of the high fusing temperature metal particles in the base material composition. The wax binder is volatile and vaporizes at a temperature during heat treatment below the melting temperature of the low fusing temperature metal particles. The filler material layers "f" of Figure 1 impregnate the base material layers "b" during heat treatment forming a unified single solidified dental coping 15 when removed from the die 10.

During heat treatment the particles of the low fusing temperature metal in the filler layer(s) "f" must melt and diffuse into the base material layer(s) "b" to cause the base material layers "b" to converge. Moreover, this must occur without any significant shrinkage in the structure 15 so that the structure 15 has a shape closely approximating the molded shape of the dental material 12 before heat treatment. This is accomplished in accordance with the present invention by selecting a thickness and binder concentration for each layer such that the layer(s) of filler material "f" will impregnate the layer(s) of base material "b" in one heat treatment operation. The variables affecting the degree of diffusion and impregnation depend upon thickness, material composition and binder concentration in the layers. The concentration of the filler material separating said layers of base material must be sufficient by volume to permit diffusion of the melted filler particles into the base material layers upon heat treatment. If insufficient concentration of filler material exists the void volume of the base material will not be completely filled up during heat treatment leaving a structure which is still porous. Alternatively excess concentration of filler material will result in a structure having bands or layers of solidified filler material. Both situations can be used advantageously in accordance with the present invention by selecting specified filler material layers "f" in the laminated arrangement to have either insufficient or excess concentrations of filler material by volume relative to the volume of adjacent layers of base material. The concentration of filler material is varied by the thickness of the layer of filler material and the concentration of binder in the filler material layer.

The relative thickness of the adjacent base material layer(s) and the concentration of binder in the base material layer(s) affect the diffusion of filler material by controlling the porosity of the base layer upon heat treatment, i.e., its void volume and capacity to be impregnated with filler material. Accordingly, the thickness and concentration of binder in the base material layers "b" can be varied to accommodate the diffusion of the filler material particularly so that only one heat treatment firing is necessary to cause the base layer "b" to be infiltrated with filler material and to solidify without shrinkage.

In addition to varying the thickness of the layers and the concentration of binder it should be understood that neither the base layers "b" nor the filler layers "f" need be of identical composition. In fact, the composition of the base layer "b" and the composition of filler layers "f" may vary to enhance the properties of the heat treated coping 15 as well as to cause the outer and/or inner surface of the structure 15 to be different in composition and density.

Moreover, although only one heat treatment operation is required it can be performed at a single temperature setting or using more than one temperature setting and in staged succession which can either be manual or automated to control the diffusion and impregnation of the low fusing metal particles in the layers "f" into the high fusing temperature layers "b".

The molding of the dental material 12 on the die 10 is explained in US Patent No. 5,593,305 as well as in US Patent No. 5,730,600. However, it should be understood that in accordance with the present invention a porous body is formed from the "b" layer(s) of high fusing metal composition and is impregnated and densified with filler material from the layer(s) "f" to form a solid structure 15. This occurs substantially simultaneously in a single heat treatment although gradual or successive firing stages may be needed during heat treatment.

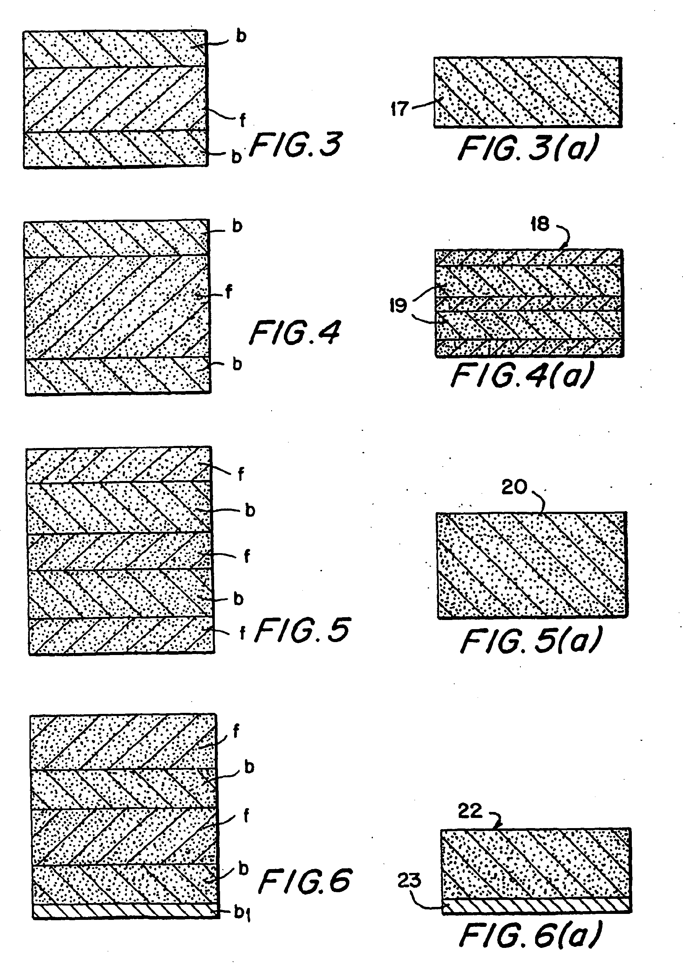

Figures 3 through 6 illustrate alternative embodiments of the invention. Figure 3 shows a three layer laminated body with an outer and inner layer of base material "b" respectively. The thickness of the filler material layer "f" is selected to provide a volume of filler material which will cause complete diffusion of the filler material into the base material layers "b" for forming a single solidified body 17 as shown in figure 3a. Figure 4 also shows a three layer laminated body with an outer and inner layer of base material "b" separated by a much thicker layer of filler material "f". In the arrangement of Figure 4 an excess of filler material by volume exists resulting in a solidified structure 18 as shown in Figure 4a having layers or bands 19 of solidified filler material following heat treatment. Alternatively, if insufficient filler material existed a solidified body would have been formed with substantially greater porosity than the structure 17 of Figure 3a. However, an unfilled or partially filled base material layer "b" may be desirable to render the outer and inner surfaces of the coping more adaptable for porcelain firing and for cementation. Figure 5 shows an arrangement of five alternating layers with the outer and inner layers composed of a filler material composition "f". In this arrangement the diffusion of filler material into the base material layers "b" are controlled through opposite adjacent sides of the base material layer "b" to cause more uniform diffusion of filler material in forming the solidified structure 20 as shown in Figure 5a. Figure 6 is yet an alternative arrangement of a five layer laminated body having two base material layers "b' and two filler material layers "f" and a fifth layer of a different base material composition "b1" from the other base material layers "b". This different base material composition "b1" may include different size high fusing temperature metal particles from that in layers "b" so as to produce a roughened surface for cementation and/or may include additional metallic constituents to achieve this objective. The solidified structure 22 as shown in Figure 6a is formed with a surface 23 having different physical characteristics from the physical characteristics in the rest of the solidified structure 22.

The dental material 12 may be formed into a laminated body in any conventional fashion such as by cladding one layer upon another with the laminated body compressed into a compacted strip of any desired geometry. During this procedure, especially when the layers are cladded and compressed, particles from one type of layer or from each type of layer may infiltrate into the other during compression i.e., particles from the "f" layers for example, may be physically incorporated into the "b" layers and vice versa particularly at their borders making it difficult to distinguish the different layers of the laminated structure from one another. Stated otherwise the border between the layers become "fuzzy". It should, however, be understood that the layers may always be distinguished from one another by analyzing their major constituents.

The thickness of the compressed laminated body may lie between 50 and 2000 µm, depending upon the specific application, with a thickness of between 100 to 500 µm preferred when forming a dental coping.

It should further be understood that the dental material of the present invention although primarily intended for forming a dental coping may also be used for repair work, to join restorations at the interproximal and to form a bridge structure.

## Claims

1. A dental material for forming a dental coping or for repair work, to join restorations at the interproximal and to form a bridge structure, said dental material being composed of a laminated structure of two or more layers including at least one layer composed of a base material composition containing high fusing temperature metal particles and a volatile wax binder in a concentration between 20% and 80% by volume of the base material composition and at least a second layer comprising a filler material composition of low fusing temperature metal particles, **characterized in that** said layers are superimposed in a tandem arrangement with each layer lying adjacent to one another in an alternating sequence and with thickness and binder concentration for each layer of base material and thickness and concentration for each layer of filler material being selected such that said layer(s) of filler material will impregnate the layer(s) of base material in one heat treatment operation at a temperature at least equal to the melting temperature of the low fusing temperature metal particles but below the melting temperature of the high fusing temperature metal particles.

2. A dental material as defined in claim 1 wherein said layer(s) of filler material have a thickness relative to the thickness of the base material composition which provides a concentration of filler material by volume such that during heat treatment essentially all of the layer(s) of filler material diffuse into the layer(s) of base material leaving only layer(s) of base material with the filler material uniformly diffused in the base material.

3. A dental material as defined in one of claims 1 or 2 wherein said high fusing temperature metal particles in each layer of base material composition comprises precious metals and alloys thereof selected from the group consisting of platinum and palladium in a proportion relative to one another of from zero to 100% alone or in combination with a precious metal or precious metal alloy selected from the group consisting of gold, silver, copper, magnesium, aluminum, zinc, gallium, indium and other metals from the third, fourth or fifth group of elements of the periodic table.

4. A dental material as defined in one of claims 1 to 3 wherein each layer of said low fusing temperature metal particles comprises gold or a gold alloy as the predominant or major constituent thereof.

5. A dental material as defined in any of the preceding claims wherein said laminated structure comprises at least three layers **characterized by** at least one layer of said base material composition separated by at least two layers of said filler material composition.

6. A dental material as defined in claim 5 wherein said laminated structure comprises at least four alternating layers arranged in tandem with two layers of base material composition.

7. A dental material as defined in claim 6 wherein said laminated structure comprises a fifth layer of a different base material composition.

8. A dental material as defined in claim 6 or claim 7 wherein said laminated structure comprises at least five alternating layers arranged in tandem wherein the fifth layer is an outer layer of filler material composition.

9. A dental material as defined in any of the preceding claims wherein said laminated structure comprises compressed multiple layers forming indistinct borders between at least some of the layers before being fired in a furnace.

10. A method for forming a dental coping comprising the steps of forming a laminated structure of two or more layers having at least one layer composed of a base material composition containing high fusing temperature metal particles and a volatile wax binder in a concentration between 20% and 80% by volume of the base material composition and at least a second layer comprising a filler material composition of low fusing temperature metal particles with the layers being superimposed in a tandem arrangement with each layer lying adjacent to one another in an alternating sequence **characterized by** the steps of adapting the laminated structure of alternating layers to a die with thickness and binder concentration for each layer of base material and thickness and concentration for each layer of filler material being selected such that said layer(s) of filler material will impregnate the layer(s) of base material in one heat treatment operation at a temperature at least equal to the melting temperature of the low fusing temperature metal particles but below the melting temperature of the high fusing temperature metal particles, and heat treating said laminated structure accordingly.

11. A method as defined in claim 10 wherein said layer(s) of filler material have a thickness relative to the thickness of the base material composition which provides a concentration of filler material by volume such that during heat treatment essentially all of the layer(s) of filler material diffuse into the layer(s) of base material leaving only layer(s) of base material with the filler material uniformly diffused in the base material.

12. A method as defined in one of claims 10 or 11 wherein the temperature of said heat treatment increases in stages.

13. A method as defined in claim 12 wherein the laminated structure comprises at least two layers of base material composition and at least two layers of filler material composition wherein the concentration of filler material in the layers of filler material composition is in excess of the void volume formed in the base layer(s) during heat treatment of the laminated structure such that the structure of the coping formed following heat treatment is non-homogeneous in density and contains bands having different void volume percentages.

## Patentansprüche

1. Dentalmaterial zur Herstellung einer Zahnhülse für Reparaturarbeiten, zur Verbindung von Restaurationen im Bereich zwischen den Approximalflächen und zur Herstellung einer Brückenstruktur, wobei das Dentalmaterial aus einer schichtartigen Struktur von zwei oder mehr Schichten besteht, die wenigstens eine Schicht, die aus einer Basismaterialzusammensetzung, die Metallpartikel mit einer hohen Schmelztemperatur und ein flüchtiges Wachsbindemittel in einer Konzentration zwischen 20 und 80 Vol.-%, bezogen auf die Basismaterialzusammensetzung enthält, und wenigstens eine zweite Schicht, die eine Füllmaterialzusammensetzung aus Metallpartikeln mit niedriger Schmelztemperatur enthält, umfasst, **dadurch gekennzeichnet, dass** die Schichten in einer tandemartigen Anordnung übereinander gelagert sind, wobei jede Schicht in einer abwechselnden Folge an eine andere grenzt und wobei die Dicke und die Bindemittelkonzentration jeder Schicht des Grundmaterials und die Dicke und Konzentration jeder Schicht des Füllmaterials so gewählt sind, dass die Schicht(en) des Füllmaterials die Schicht(en) des Basismaterials während einer Wärmebehandlung bei einer Temperatur, die wenigstens so hoch wie die Schmelztemperatur der bei niedriger Temperatur schmelzenden Metallpartikel, aber geringer als die Schmelztemperatur der bei hoher Temperatur schmelzenden Metallpartikel ist, imprägniert (imprägnieren).

2. Dentalmaterial gemäß Anspruch 1, wobei die Schicht(en) des Füllmaterials eine Dicke bezogen auf die Dicke der Basismaterialzusammensetzung hat (haben), die für eine solche Volumenkonzentration des Füllmaterials sorgt, dass während der Wärmebehandlung im wesentlichen die gesamte(n) Schicht(en) des Füllmaterials in die Schicht(en) des Basismaterials hinein diffundiert (diffundieren) und nur eine Schicht (Schichten) des Basismaterials resultiert (resultieren), bei denen das Füllmaterial gleichmäßig im Basismaterial verteilt ist.

3. Dentalmaterial gemäß einem der Ansprüche 1 oder 2, wobei die bei hoher Temperatur schmelzenden Metallpartikel in jeder Schicht der Basismaterialzusammensetzung Edelmetalle und deren Legierungen umfassen, die ausgewählt sind aus der Gruppe bestehend aus Platin und Palladium in einem relativen Anteil zueinander, der im Bereich von 0 bis 100% liegt, allein oder in Kombination mit einem Edelmetall oder einer Edelmetalllegierung, die ausgewählt ist aus der Gruppe bestehend aus Gold, Silber, Kupfer, Magnesium, Aluminium, Zink, Gallium, Indium und anderen Metallen der dritten, vierten oder fünften Gruppe des Periodensystems der Elemente.

4. Dentalmaterial gemäß einem der Ansprüche 1 bis 3, wobei die Schicht der bei niedriger Temperatur schmelzenden Metallpartikel Gold oder eine Goldlegierung als vorherrschenden oder hauptsächlichen Bestandteil umfasst.

5. Dentalmaterial gemäß einem der vorhergehenden Ansprüche, wobei die schichtartige Struktur wenigstens drei Schichten umfasst, **gekennzeichnet durch** wenigstens eine Schicht der Basismaterialzusammensetzung, die **durch** wenigstens zwei Schichten der Füllmaterialzusammensetzung getrennt ist.

6. Dentalmaterial gemäß Anspruch 5, wobei die schichtartige Struktur wenigstens vier abwechselnde Schichten umfasst, die tandemartig mit zwei Schichten der Basismaterialzusammensetzung angeordnet sind.

7. Dentalmaterial gemäß Anspruch 6, wobei die schichtartige Struktur eine fünfte Schicht aus einer unterschiedlichen Basismaterialzusammensetzung umfasst.

8. Dentalmaterial gemäß Anspruch 6 oder Anspruch 7, wobei die schichtartige Struktur wenigstens fünf abwechselnde Schichten umfasst, die tandemartig angeordnet sind, wobei die fünfte Schicht eine äußere Schicht der Füllmaterialzusammensetzung ist.

9. Dentalmaterial gemäß einem der vorhergehenden Ansprüche, wobei die schichtartige Struktur verdichtete Vielfachschichten umfasst, welche undeutliche Grenzen zwischen wenigstens einigen der Schichten bilden, bevor sie in einem Ofen gebrannt werden.

10. Verfahren zur Herstellung einer Dentalhülse, umfassend die Schritte: Bilden einer schichtartigen Struktur von zwei oder mehr Schichten, wobei wenigstens eine Schicht aus einer Basismaterialzusammensetzung, die bei hoher Temperatur schmelzenden Metallpartikel und ein flüchtiges Wachsbindemittel in einer Konzentration zwischen 20 und 80 Vol.-%, bezogen auf die Basismaterialzusammensetzung, enthält, und wenigstens eine zweite Schicht aufweist, die eine Füllmaterialzusammensetzung aus bei niedriger Temperatur schmelzenden Metallpartikeln umfasst, wobei die Schichten in einer tandemartigen Anordnung übereinander angeordnet sind, bei der jede Schicht in einer abwechselnden Folge an eine andere Schicht grenzt, **gekennzeichnet durch** das Anpassen der schichtartigen Struktur aus abwechselnden Schichten an eine Form, wobei die Dicke und Bindemittelkonzentration für jede Schicht des Basismaterials und die Dicke und Konzentration jeder Schicht des Füllmaterials so gewählt sind, dass die Schicht(en) des Füllmaterials die Schicht(en) des Basismaterials bei einer Wärmebehandlung bei einer Temperatur, die wenigstens gleich der Schmelztemperatur der bei niedriger Temperatur schmelzenden Metallpartikel, aber niedriger als die Schmelztemperatur der bei hoher Temperatur schmelzenden Metallpartikel ist, imprägniert (imprägnieren) und entsprechendes Wärmebehandeln der schichtartigen Struktur.

11. Verfahren gemäß Anspruch 10, wobei die Schicht(en) des Füllmaterials eine Dicke bezogen auf die Dicke der Basismaterialzusammensetzung aufweist, die für eine solche Volumenkonzentration des Füllmaterials sorgt, dass bei der Wärmebehandlung im wesentlichen jede Schicht(en) des Füllmaterials in die Schicht(en) des Basismaterials hinein diffundiert (diffundieren) und nur eine Schicht (Schichten) des Basismaterials resultiert (resultieren), bei der (denen) das Füllmaterial gleichmäßig in dem Basismaterial verteilt ist.

12. Verfahren gemäß einem der Ansprüche 10 oder 11, bei dem die Temperatur der Wärmebehandlung stufenartig ansteigt.

13. Verfahren gemäß Anspruch 12, wobei die schichtartige Struktur wenigstens zwei Schichten der Basismaterialzusammensetzung und wenigstens zwei Schichten der Füllmaterialzusammensetzung umfasst, wobei die Konzentration des Füllmaterials in den Schichten der Füllmaterialzusammensetzung das freie Volumen übersteigt, das während der Wärmebehandlung der schichtartigen Struktur in der Basisschicht (den Basisschichten) gebildet wird, so dass die Struktur der im Anschluss an die Wärmebehandlung gebildeten Hülse eine inhomogene Dichte aufweist und Bänder enthält, die unterschiedliche prozentuale Leervolumina aufweisen.

## Revendications

1. Matériau dentaire pour former une coiffe dentaire ou pour effectuer un travail de réparation, afin de relier des restaurations au niveau interproximal et former une structure de bridge, ledit matériau dentaire étant composé d'une structure stratifiée de deux couches ou plus comprenant au moins une couche composée d'une composition de matière de base contenant des particules métalliques à haute température de fusion et un liant cireux volatil en une concentration comprise entre 20% et 80% en volume de la composition de matière de base, et au moins une deuxième couche comprenant une composition de matière de charge de particules métalliques à basse température de fusion, **caractérisé en ce que** lesdites couches sont superposées selon une disposition en tandem dans laquelle chaque couche est adjacente à l'autre selon une séquence alternée, et l'épaisseur et la concentration de liant pour chaque couche de matière de base et l'épaisseur et la concentration pour chaque couche de matière de charge étant choisies de sorte que ladite ou lesdites couches de matière de charge imprègnent la ou les couches de matière de base au cours d'une opération de traitement thermique, à une température au moins égale à la température de fusion des particules métalliques à basse température de fusion mais inférieure à la température de fusion des particules métalliques à haute température de fusion.

2. Matériau dentaire tel que défini dans la revendication 1, dans lequel ladite ou lesdites couches de matière de charge ont une épaisseur par rapport à l'épaisseur de la composition de matière de base qui permet d'obtenir une concentration de matière de charge en volume telle que, pendant le traitement thermique, la quasi-totalité de la ou des couches de matière de charge se diffuse dans la ou les couches de matière de base, laissant uniquement une ou des couches de matière de base avec la matière de charge diffusée de manière uniforme dans la matière de base.

3. Matériau dentaire tel que défini dans l'une des revendications 1 ou 2, dans lequel lesdites particules métalliques à haute température de fusion dans chaque couche de composition de matière de base comprennent des métaux précieux et des alliages de ceux-ci choisis dans le groupe constitué par le platine et le palladium, dans une proportion relative l'un par rapport à l'autre de zéro à 100%, seuls ou en combinaison avec un métal précieux ou un alliage de métal précieux choisi dans le groupe constitué par l'or, l'argent, le cuivre, le magnésium, l'aluminium, le zinc, le gallium, l'indium et d'autres métaux du troisième, quatrième ou cinquième groupe d'éléments de la classification périodique des éléments.

4. Matériau dentaire tel que défini dans l'une des revendications 1 à 3, dans lequel chaque couche desdites particules métalliques à basse température de fusion comprend de l'or ou un alliage d'or en tant que constituant prédominant ou majeur de celles-ci.

5. Matériau dentaire tel que défini dans l'une quelconque des revendications précédentes, dans lequel ladite structure stratifiée comprend au moins trois couches **caractérisées par** au moins une couche de ladite composition de matière de base séparée par au moins deux couches de ladite composition de matière de charge.

6. Matériau dentaire tel que défini dans la revendication 5, dans lequel ladite structure stratifiée comprend au moins quatre couches alternées disposées en tandem avec deux couches de composition de matière de base.

7. Matériau dentaire tel que défini dans la revendication 6, dans lequel ladite structure stratifiée comprend une cinquième couche d'une composition différente de matière de base.

8. Matériau dentaire tel que défini dans la revendication 6 ou la revendication 7, dans lequel ladite structure stratifiée comprend au moins cinq couches alternées disposées en tandem, la cinquième couche étant une couche externe de composition de matière de charge.

9. Matériau dentaire tel que défini dans l'une quelconque des revendications précédentes, dans lequel ladite structure stratifiée comprend plusieurs couches comprimées formant des frontières indistinctes entre au moins certaines des couches avant d'être cuite dans un four.

10. Procédé pour former une coiffe dentaire comprenant les étapes consistant à former une structure stratifiée de deux couches ou plus comprenant au moins une couche composée d'une composition de matière de base contenant des particules métalliques à haute température de fusion et un liant cireux volatil en une concentration comprise entre 20% et 80% en volume de la composition de matière de base, et au moins une deuxième couche comprenant une composition de matière de charge de particules métalliques à basse température de fusion, les couches étant superposées selon une disposition en tandem dans laquelle chaque couche est adjacente à l'autre selon une séquence alternée, **caractérisé par** les étapes consistant à adapter la structure stratifiée de couches alternées à un modèle, l'épaisseur et la concentration de liant pour chaque couche de matière de base et l'épaisseur et la concentration pour chaque couche de matière de charge étant choisies de sorte que ladite ou lesdites couches de matière de charge imprègnent la ou les couches de matière de base au cours d'une opération de traitement thermique, à une température au moins égale à la température de fusion des particules métalliques à basse température de fusion mais inférieure à la température de fusion des particules métalliques à haute température de fusion, et à traiter thermiquement ladite structure stratifiée de manière appropriée.

11. Procédé tel que défini dans la revendication 10, dans lequel ladite ou lesdites couches de matière de charge ont une épaisseur par rapport à l'épaisseur de la composition de matière de base qui permet d'obtenir une concentration de matière de charge en volume telle que, pendant le traitement thermique, la quasi-totalité de la ou des couches de matière de charge se diffuse dans la ou les couches de matière de base, laissant uniquement une ou des couches de matière de base avec la matière de charge diffusée de manière uniforme dans la matière de base.

12. Procédé tel que défini dans l'une des revendications 10 ou 11, dans lequel la température dudit traitement thermique augmente par étapes.

13. Procédé tel que défini dans la revendication 12, dans lequel la structure stratifiée comprend au moins deux couches de composition de matière de base et au moins deux couches de composition de matière de charge, la concentration de matière de charge dans les couches de composition de matière de charge étant supérieure au volume de vides formés dans la ou les couches de base pendant le traitement thermique de la structure stratifiée, de sorte que la structure de la coiffe formée à la suite du traitement thermique a une densité non homogène et contient des bandes ayant différents pourcentages de volume de vides.
